# EUROPÄISCHE PATENTSCHRIFT

(11) **EP 2 659 846 B1**
(45) Veröffentlichungstag und Bekanntmachung des Hinweises auf die Patenterteilung: **13.12.2017**
(21) Anmeldenummer: 12166344.7
(22) Anmeldetag: 02.05.2012
(51) Int. Cl.: A61B 18/00, A61B 18/04, A61B 18/12, A61B 18/14

(54) **Elektrochirurgische Einrichtung mit Mitteln zur Erzeugung einer Lichterscheinung und zur Gewebeunterscheidung durch Lichtanalyse**
Electrosurgical apparatus with means for producing a light appearance and for differentiating between tissues through light analysis
Appareil électrochirurgical avec des moyens pour créer une apparition de lumière et pour différencier des tissus par l'analyse de la lumière

(43) Veröffentlichungstag der Anmeldung: 06.11.2013
(60) Teilanmeldung: 17192484.8
(73) Patentinhaber: Erbe Elektromedizin GmbH, 72072 Tübingen (DE)
(72) Erfinder: Neugebauer, Alexander, 72116 Mössingen (DE); Fischer, Klaus, 721202 Nagold (DE); Enderle, M., 72070 Tübingen (DE)
(74) Vertreter: Rüger, Barthelt & Abel

(56) Entgegenhaltungen:
- EP-A2- 2 392 278
- WO-A2-2011/055369
- US-A1- 2007 213 704
- US-A1- 2009 326 383
- US-A1- 2012 035 608

## Beschreibung

Die Erfindung betrifft eine chirurgische Einrichtung zur Vornahme von chirurgischen Untersuchungen und/oder Eingriffen mit Mitteln zur Gewebeunterscheidung.

Patienten werden im Allgemeinen nach erfolgter Diagnose und Feststellung der Indikation einer Operation unterzogen. Wenn zur Planung der Operation eine Gewebeanalyse erforderlich ist, wird diese in der Regel vor der Operation in einer separaten Sitzung durch Biopsie und Analyse des Biopsats in einem pathologischen Labor durchgeführt. Während der Operation sind Gewebeanalysen aber nur sehr eingeschränkt möglich. Wird eine pathologische Analyse noch im Operationssaal durchgeführt, ist dieses materiell und zeitlich aufwendig und wird deshalb nur sehr selten praktiziert. Ebenso sind Untersuchungen von Gewebeproben im pathologischen Labor aufwendig, so dass sie während der Operation nur sehr eingeschränkt durchgeführt werden können.

Es ist zu wünschen, eine Möglichkeit zu schaffen, bei der direkt an einem Patienten, beispielsweise während eines elektrochirurgischen Eingriffs, eine Gewebeanalyse schnell und mit hoher Aussagekraft durchgeführt werden kann. Es wäre besonders zu wünschen, wenn die Möglichkeit der Differenzierung von Geweben während einer laufenden Operation gegeben wäre.

Aus der DE 103 92 791 T5 ist eine endoskopische Einrichtung bekannt, die eine HF-Ablationsvorrichtung sowie einen Kohärenz-Reflektionsmesser aufweist. Mittels der HF-Ablationsvorrichtung kann Gewebe HF-chirurgisch behandelt werden. Zu dem Kohärenz-Reflektionsmesser gehört ein Lichtleiter, über den Licht auf das zu analysierende Gewebe gestrahlt und reflektiertes Licht zu einer Analyseeinrichtung zurück geleitet wird. Diese nutzt zur Unterscheidung von gesundem und krankem Gewebe die unterschiedlichen Streuintensitäten, die zum Beispiel eine arterielle Wand von der Umgebung unterscheiden.

Weiter ist aus der DE 198 60 689 C2 eine HF-chirurgische Einrichtung mit einer Temperaturabtasteinrichtung bekannt, die an dem Instrument vorgesehen ist und zum Beispiel als Pyrometer oder auch als Empfänger für sichtbares Licht ausgebildet ist. Die Temperaturabtasteinrichtung dient zur Erfassung der Temperatur des Behandlungsbereichs oder der Lichtemission in diesem Bereich. Das von der Temperaturabtasteinrichtung gelieferte Ausgangssignal wird einer Vergleichereinrichtung zugeführt, in der es mit einem voreinstellbaren Wert verglichen werden kann, so dass bei Überschreiten dieses voreingestellten Werts ein Steuersignal erzeugt wird. Dieses kennzeichnet einen Betriebszustand, in welchem die Temperatur des Behandlungsbereichs so hoch ist, dass Rauch entstehen kann. In Abhängigkeit davon kann eine Absaugeinrichtung eingeschaltet werden. Daraus folgt eine Minimierung des Absaugvorgangs mit den damit verbundenen Störungen bei gleichzeitiger Beibehaltung oder sogar Steigerung der Absaugeffizienz.

Aus der US 2007/0213704 A1 ist ein medizinisches Instrument zur Kaltabtragung von Gewebe durch extrem kurze HF-Funken bekannt. Das von den Funken ausgehende Licht wird einem Spektralanalysator zugeführt, um anhand der erfassten Spektren Steuersignale zu erzeugen und eine Gewebeunterscheidung zu ermöglichen.

Weiter ist aus der WO 2011/055369 A2 ein Katheter zur Plaqueabtragung aus Blutgefäßen bekannt. Der zur Abtragung genutzte Funken erzeugt Licht, das über eine Lichtleitfaser einem Sensor zugeführt wird. Dieser erfasst die Phosphorlinie, um Plaques von lebenden Zellen zu unterscheiden.

Aus der US 2009/0326383 A1 ist ein Verfahren zur Erkennung von malignen Hautveränderungen bekannt, wobei dazu eine Hyperspektralanalyse des von dem Patienten reflektierten Lichts genutzt wird. Unter "Licht" wird bei der Hyperspektralanalyse ein breiter Wellenlängenbereich verstanden, der vom Terahertzbereich bis in den Röntgenbereich reicht.

Auf dem Gebiet des Kunststoffrecyclings schlägt die DE 42 31 677 A1 den Einsatz von Gleitfunkenentladungen vor, die durch eine niederfrequente Folge von Hochspannungs/ Hochstrom-Impulsentladungen erzeugt werden. In den Gleitfunkenentladungen entsteht ein Lichtbogenplasma, das die Elektroden aufheizt, um eine thermische Elektronenemission zu erreichen. So wird auf der Oberfläche nichtleitender Kunststoffe ein sogenannter Gleitfunken mit einem Lichtbogenplasma erzeugt, das im Wesentlichen im thermischen Gleichgewicht steht (Elektronentemperatur stimmt im Wesentlichen mit der Temperatur der Atome und Ionen überein). Das Lichtbogenplasma ist so heiß, dass sowohl an der Oberfläche des nichtleitenden Kunststoffs als auch an den Elektroden ein Materialabtrag entsteht und chemische Bindungen im verdampften Material aufgebrochen werden. In dem Lichtbogen werden die atomaren Bestandteile des erhaltenen Plasmas zum Leuchten angeregt. Z.B. entsteht beim Verdampfen von PVC (Polyvinylchlorid) aggressives Chlor, das die verwendeten aufgeheizten Kupferelektroden angreift, den Kupferabtrag erhöht und den Lichtbogen grün färbt. Durch Spektralanalyse des von dem Lichtbogen des Gleitfunkens ausgesandten Lichts kann auf die Art des Kunststoffs geschlossen werden.

Diese Vorgehensweise ist zur Anwendung am lebenden Gewebe ungeeignet.

Es ist Aufgabe der Erfindung, eine Einrichtung zu schaffen, die in biologischem Gewebe eine Gewebeunterscheidung ermöglicht.

Diese Aufgabe wird mit einer Einrichtung nach Anspruch 1 gelöst.

Zu der erfindungsgemäßen Einrichtung gehört ein Instrument zur elektrochirurgischen Einwirkung auf ein biologisches Gewebe unter Erzeugung einer Lichterscheinung, wobei dieses Instrument dazu mindestens eine mit elektrischer HF-Energie gespeiste Elektrode aufweist. Die HF-Funkenentladung ist durch eine hochfrequente Spannung gespeist, die vorzugsweise eine Leerlaufspannung zwischen 100 und 6000 V aufweist. Die Frequenz der hochfrequenten Spannung liegt vorzugsweise zwischen 100 kHz und 5 MHz oder wenigstens in der Nähe dieses Bereichs. Die Frequenz und Wellenform sind zur Vermeidung oder Minimierung neuromuskulärer Reize gestaltet. Die HF-Spannung ist vorzugsweise eine kontinuierlich anliegende oder eine gepulste Sinuswelle. Der Scheitelwert des Stroms des HF-Funkens beträgt vorzugsweise höchstens 6 A, vorzugsweise ist er geringer als dieser Wert. Vorzugsweise wird der HF-Funken so gesteuert und/oder ausgebildet, dass er ein im thermischen Ungleichgewicht stehendes Plasma ausbildet. Bei diesem ist die Elektronentemperatur größer, vorzugsweise erheblich größer, als die nachfolgend als "Plasmatemperatur" bezeichnete Temperatur der im Plasma enthaltenen Moleküle, Molekülbruchstücke, Radikale, Atome und/oder Ionen.

Der HF-Funken bildet sich z.B. zwischen der Elektrode und dem biologischen Gewebe aus und wird ausgehend von der relativ kleinflächigen Elektrode eines monopolaren chirurgischen Instruments durch das biologische Gewebe (z.B. das Gewebe eines lebenden Patienten hindurch) zu einer an dem Gewebe (z.B. an einer Körperfläche des Patienten) angebrachten Neutralelektrode geführt. Die Elektrode des chirurgischen Instruments ist so ausgebildet, dass sie gegenüber dem HF-Funken stabil ist, d.h. in Betrieb keinen relevanten Materialabtrag aufweist. Außerdem ist sie aus einem physiologisch unbedenklichen Material, wie z.B. Wolfram, Titan, Edelstahl oder elektrisch leitfähiger Keramik ausgebildet.

Alternativ kann der Funken bei einem Plasmastrahlinstrument auch in einem Gasstrom, beispielsweise einem Argonstrom, brennen, wodurch ein auf das biologische Gewebe strömendes Plasma erzeugt wird, von dem wiederum eine Lichterscheinung ausgeht. Vorzugsweise handelt es sich auch hier um ein im thermischen Ungleichgewicht stehendes Plasma, bei dem die Elektronentemperatur größer, vorzugsweise erheblich größer ist, als die Plasmatemperatur.

In beiden Fällen, d.h. sowohl bei dem monopolaren Instrument als auch bei dem Plasmastrahlinstrument, wird die entstehende Lichterscheinung von dem biologischen Gewebe beeinflusst, das mit dem entstehenden Plasma interagiert. Die Plasmatemperatur wird dabei so niedrig gehalten, dass in dem Plasma verdampfte Teile des biologischen Gewebes nicht vollständig dissoziiert sind. Die entstehende "Lichterscheinung" bzw. "optische Emission" ist nicht auf das vom menschlichen Auge erfassbare Spektrum beschränkt. Sie kann auch ganz oder teilweise im unsichtbaren, d.h. z.B. ultravioletten und infraroten Spektralbereich liegen oder in mindestens einen dieser Spektralbereiche hineinreichen.

Ein Einfluss der Elektrode auf die Lichterscheinung wird durch Vermeidung relevanten Materialabtrags von der Elektrode vermieden oder zumindest reduziert. Das von dem Plasma ausgehende Licht wird aufgenommen und einer Analyse oder Einrichtung zur Bestimmung von Lichtmerkmalen zugeleitet. Die Lichtmerkmale können im weitesten Sinne die spektrale Zusammensetzung des Lichts betreffen. Zur Erfassung dieser Lichtmerkmale ist eine Diskriminatoreinrichtung vorgesehen. Diese dient der Zuordnung von Lichtmerkmalen zu Gewebemerkmalen.

Bei den Lichtmerkmalen kann es sich um:
typische Spektren,
Ausschnitte von Spektren,
aus Spektren gewonnene Signaturen,
Intensitäten einzelner Spektrallinien
Intensitäten von Spektralliniengruppen und/oder
Signaturen handeln, die aus:
   dem Spektrum des Lichts des Plasmas,
   Ausschnitten des Spektrums und/oder
   Spektrallinien gewonnen sind.

Signaturen können als Rechengrößen aus Algorithmen, Formeln, Zuordnungstabellen oder sonstigen Verarbeitungsvorschriften gewonnen werden. Insbesondere können Signaturen:
Intensitätsverhältnisse ausgewählter Spektrallinien, Verhältnisse, Summen und/oder Differenzen zwischen verschiedenen Termen sein, wobei die Terme ihrerseits wiederum:
   Summen,
   Differenzen,
   Produkte,
   Integrale über Teilspektren und/oder
      andere Terme von Intensitäten verschiedener Spektrallinien oder Teilspektren sind.

Mit diesen Auswertemaßnahmen ist die Unterscheidung verschiedener Gewebe, wie bspw. Muskelgewebe, Fettgewebe, Blut, Knochengewebe, Bindegewebe, Knorpel sowie die Unterscheidung zwischen gesundem Gewebe und pathologischem Gewebe, wie z.B. Tumoren möglich. Die Unterscheidung kann anhand von in einer Datenbank abgelegten Lichtmerkmalen erfolgen, die zu bestimmten Geweben gehören und die mit den Merkmalen des aufgenommenen Lichts verglichen werden. Es ist möglich, eine Datenbank vorzusehen, in der Lichtmerkmale typischer Gewebe vorhanden sind. Es ist auch möglich, einen Kanal vorzusehen, auf dem individuelle Lichtmerkmale von bestimmten Geweben eines Patienten in die Datenbank geleitet werden, wobei diese Lichtmerkmale vor oder während der Operation aufgenommen worden sein können.

Zu der Einrichtung gehört ein Instrument, das ein auf das biologische Gewebe einwirkendes Plasma bereitstellt. Es kann z.B. mindestens eine HF-Elektrode aufweisen, die über eine Leitung mit dem speisenden HF-chirurgischen Gerät verbunden ist und so mit HF-Leistung versorgt wird. Dem Instrument sind weiterhin mehrere Lichtaufnahmeeinrichtungen zugeordnet, die jeweils auf einen Teilbereich des die Lichterscheinung enthaltenden Bereichs ausgerichtet sind. Die Lichtaufnahmeeinrichtungen können bspw. direkt und fest mit dem Instrument verbunden sein. Es ist auch möglich, diese oder eine andere, z.B. eine weitere Lichtaufnahmeeinrichtung von dem Instrument gesondert bereitzustellen und so auf das Operationsgebiet auszurichten, dass sie das dort entstehende Licht aufnimmt.

Die Lichtaufnahmeeinrichtungen nehmen vorwiegend Licht aus unterschiedlichen Abschnitten des die Lichterscheinung enthaltenden Bereichs auf. Damit wird es möglich, Richtungsinformation zu gewinnen. Wenn beispielsweise ein HF-Funke an einer Seite einer Elektrode ein bestimmtes (z.B. ein malignes) Gewebe berührt und verdampft, während auf der anderen Seite der Elektrode ein anderes (z.B. nicht malignes, gesundes) Gewebe ansteht, kann auf diese Weise unterschieden werden, auf welcher Seite der Elektrode Gewebe eines bestimmten Typs (gesund oder krank, Fett oder Muskel o.ä.) ansteht. Eine solche Unterscheidung ist insbesondere möglich, wenn die Lichtaufnahmeeinrichtung jeweils lediglich auf einen Teilbereich der Lichterscheinung ausgerichtet ist.

Vorzugsweise ist das Instrument auf eine Operationsfeldbeleuchtung abgestimmt. Die Analyseeinrichtung der Lichterscheinung ist in demjenigen Spektralbereich unempfindlich, in dem die Operationsfeldbeleuchtung arbeitet. Auf diese Weise wird eine Blendung der Analyseeinrichtung und der Diskriminatoreinrichtung vermieden.

Mit der erfindungsgemäßen Einrichtung besteht die Möglichkeit der Differenzierung von Geweben während der laufenden Operation. Es ist möglich, dem Operateur Informationen darüber zu geben, welche Art Gewebe er aktuell plasmachirurgisch oder elektrochirurgisch behandelt, bzw. wie das Gewebe zusammengesetzt ist. Es ist eine Differenzierung von gesundem und pathologischem Gewebe möglich, ohne eine zeitraubende histopathologische Analyse durchführen zu müssen und ohne den Patienten im Bedarfsfall zu einer erneuten Operation einbestellen zu müssen, wenn im verbleibenden Gewebe nach wie vor pathologische Befunde vorhanden sind. Die mit Nachoperationen einhergehenden höheren Belastungen und erhöhten Risiken für den Patienten können so ebenso vermieden werden wie Zeitverzögerungen während der Operation.

In Ausgestaltung der Erfindung ist es möglich, die von der HF-Elektrode oder einem Plasmastrahl abgegebene Energie automatisch oder durch Automatismen unterstützt auf das aktuell behandelte Gewebe anzupassen. Pathologisches Gewebe kann z.B. krebsinfiltriertes oder neoplastisches Gewebe sein.

Ein immer angestrebtes Ziel bei der Entfernung lokal begrenzter Karzinome ist eine vollständige Entfernung des krebsinfiltrierten Gewebes mit tumorfreien Resektionsrändern. Dies wird als R0-Resektion bezeichnet. Diese führt zur Heilung, sofern zum Zeitpunkt der Operation keine Metastatisierung vorliegt. Kann jedoch im Absetzungsrand histologisch noch Krebsgewebe nachgewiesen werden, wird von einer R1-Resektion gesprochen, die zum Beispiel beim Prostatakarzinom mit einer Rezidivrate von 20% bis 60% verbunden ist. Auch mit größter operativer und klinischer Erfahrung des Operateurs und Verwendung einer Lupenbrille ist die Unterscheidung zwischen tumortragendem und tumorfreiem Gewebe jedoch nicht immer eindeutig möglich. Deshalb ist eine R0-Resektion mit den heute verfügbaren chirurgischen Techniken häufig nur unzureichend realisierbar. Die Erfindung weist einen Weg, wie durch Lichtanalyse des bei der Einwirkung des HF-Funkens oder eines Plasmastrahls entstehenden Lichts eine solche Unterscheidung objektiv möglich wird. Die größere Sicherheit bei der Unterscheidung von pathologischem von gesundem Gewebe gibt dem Operateur eine Möglichkeit in die Hand, eine RO-Resektion mit erhöhter Sicherheit zu erreichen. Die Einrichtung kann dabei auf verschiedene chirurgische Anwendungen angewendet werden, bei denen Lichterscheinungen auftreten, wie insbesondere HF-Schneiden, HF-Ablation, HF-Koagulation, Plasmakoagulation, Plasmaschneiden, Plasmaablation und dergleichen. Es wird dabei keine externe Lichtquelle benötigt. Vielmehr entsteht die Lichtemission während der elektrochirurgischen Anwendung selbst.

Die erfindungsgemäße chirurgische Einrichtung kann in Verbindung mit einer Markersubstanz Anwendung finden. Die Markersubstanz kann beispielsweise ein Fluoreszenzfarbstoff sein, der bei thermischer oder elektrischer Anregung im Funken oder Plasmastrahl auf mindestens einer Spektrallinie leuchtet, die sonst von biologischem Gewebe nicht oder nur schwach erzeugt wird. Die Markersubstanz kann zum Beispiel durch eine phamakophore Gruppe gebildet werden, - die zu einem Membranantigen eines Karzinoms passt und eine bei Anregung charakteristisch leuchtende Gruppe (Fluoreszenzlabel oder Emissionslabel) trägt. Am Beispiel eines Prostatakarzinoms ist dies bspw. eine phamakophore Gruppe, die zu einem prostataspezifischen Membranantigen (PSMA) passt, welches im Falle des Karzinoms viel stärker exprimiert wird als im gesunden Prostatagewebe. Das Emissionslabel kann zum Beispiel Metallatome enthalten, die eine oder mehrere charakteristische Spektrallinie(n) festlegen. Ist die Markersubstanz an der Zelloberfläche des Prostataepithels angereichert, führt dies zu einer charakteristischen Form (Spektralzusammensetzung) der von der Diskriminatoreinrichtung zu erfassenden Leuchterscheinung, sobald der HF-Funke das Prostataepithel berührt. Wird das zu erkennende Gewebe mit einer Markersubstanz markiert, wird in dem optischen Emissionsspektrum gezielt nach Signalen, d.h. mindestens einer Spektrallinie der Markersubstanz gesucht. Die Spektrallinie liegt vorzugsweise auf einer Wellenlänge, bei der bei gesundem Gewebe sonst keine nennenswerte Lichtemission auftritt.

Die Markersubstanz kann lokal, oral oder intravenös oder auf andere geeignete Weise vor dem chirurgischen Eingriff appliziert werden. Die Markersubstanz bindet selektiv mit hoher Spezifität an eine Komponente der jeweiligen Krebszelle. Beispielsweise bindet die Markersubstanz im Falle des Prostatakarzinoms an das prostataspezifische Membranantigen (PSMA). Neben der Detektion des Prostatakarzinoms über PSMA sind jedoch zahlreiche weitere Krebsarten erfassbar. Dazu zählt beispielsweise der Brustkrebs mittels des cancer antigen CA15-3 und das carzino-embrionic antigen (CEA), der Dickdarmkrebs (durch das cancer antigen 19-9), das Ovarialkarzinom (CA72-4, CA125, CASA), der Magenkrebs (CEA, CA72-4, CA19-9), der Leberkrebs (durch das Alpha-Fetoprotein (AFP)), der Ösophagus (CEA, SCC), das Chorion (hCG), Lunge SCLC (NSE, ProGRP, CYFRA21-1), Lunge NSCLC (CEA, CYFRA21-1), Keimzellen (AFP, hCG), Blase (CYFRA21-1), Schilddrüse(CEA, hTG) und malignes Melanom (S100). Die Markersubstanz besteht aus einer pharmakophoren Gruppe, welche für die Bindung an das biologische Zielgewebe zuständig ist, sowie einem Label zur Erzeugung einer optischen Emission. Als Label sind prinzipiell alle Elemente geeignet, welche bevorzugt nicht im menschlichen Organismus vorkommen und keine toxische Wirkung aufweisen.

Im Falle des Prostatakarzinoms wird das molekulare Target PSMA stärker als im Normalgewebe exprimiert. Biomarker mit hoher Selektivität gegenüber dem Zielgewebe markieren das krebstragende Gewebe sehr effektiv. Die Markersubstanz reichert sich mit hoher Selektivität im krebstragenden Gewebe an, wobei sie im krebsfreien Gewebe kaum vorhanden ist. Bei der elektrochirurgischen Anwendung führt die Markersubstanz zu einer Lichtemission, wobei die Anregungsenergie aus der elektrochirurgischen Anwendung selbst stammt. Diese kann eine kontaktlose Argon-PlasmaKoagulation sowie sämtliche Schneide-Koagulations- und Vaporisationsanwendungen mit hochfrequentem Wechselstrom sein. Die Anwendung ist nicht auf spezielle Elektrodenformen beschränkt. Es können Schlingenelektroden, Nadelelektroden, Spatelelektroden, Plasmastrahlen und dergleichen Anwendung finden.

Die Unterscheidung von Gewebetypen und Arten sowie von gesundem und krankem Gewebe ist auch ohne Verwendung von Markersubstanzen möglich. Beispielsweise besitzen unterschiedliche nichtpathologische Gewebetypen sowie pathologische Gewebe, z.B. neoplastisches Gewebe, Tumorgewebe usw., einen unterschiedlichen biochemischen Aufbau, der in der optischen Emission analysierbar wird, die bei der elektrochirurgischen Anwendung auftritt. Das entstehende (sichtbare und/oder unsichtbare) Licht enthält Lichtmerkmale, denen Gewebemerkmale zugeordnet werden können.

Die Gewebediskrimination kann durch einen Auswertealgorithmus gewonnen werden. Referenzspektren oder charakteristische Lichtmerkmale von gesundem und krankem Gewebe, verschiedenen Gewebetypen können in einer Datenbank abgelegt sein. Die Datenbank kann statisch, d.h. vor Operationsbeginn mit entsprechenden Daten gefüllt sein. Es ist auch möglich, die Daten vor oder während der Operation zu gewinnen oder nach zu justieren. Beispielsweise kann vorgesehen sein, dass der Chirurg, solange er sicher in einem bestimmten Gewebe (z.B. in gesundem Gewebe) schneidet, wenigstens ein auftretendes Lichtmerkmal als Sollgröße abspeichert. Später erkannte Abweichungen von dieser Sollgröße können von der Diskriminatoreinrichtung erfasst werden, um ein Signal zu erzeugen. Die Einrichtung kann dieses Signal weiterverarbeiten und weitergeben. Auf diese Weise können auch mehrere Lichtmerkmale gewonnen und abgespeichert werden. Allgemein kann gesagt werden, dass die Diskriminatoreinrichtung voreingestellte oder patientenindividuell gewonnene Referenzspektren oder einzelne Lichtmerkmale dieser Spektren mit einem gemessenem Spektrum oder einzelnen Lichtmerkmalen desselben vergleicht und anhand von Abweichungen Signale erzeugt, die eine Änderung der Gewebeart oder Beschaffenheit anzeigen. Patientenindividuelle Lichtmerkmale, z.B. ein patientenindividuelles Referenzspektrum, können zu Beginn einer Operation oder auch ein- oder mehrfach während der Operation aufgenommen und gespeichert werden.

Weitere Einzelheiten vorteilhafter Einrichtungen sind Gegenstand der Zeichnungen, der Beschreibung oder von Ansprüchen. Es zeigen:
Figur 1 eine Einrichtung in einer beispielhaften, schematisiert dargestellten Ausführungsform.
Figur 2 die Anwendung der Einrichtung an einem Patienten, in schematisierter Darstellung.
Figur 3 die Anwendung der Einrichtung an einem Patienten, wiederum in stark schematisierter Einrichtung.
Figur 4 eine Diskriminatoreinrichtung einer chirurgischen Einrichtung als Blockschaltbild.
Figur 5 ein Instrument zur elektrochirurgischen Behandlung, in schematisierter ausschnittsweiser Seitenansicht.
Figur 6 eine HF-Elektrode und benachbarte Beobachtungsgebiete, in schematisierter Darstellung.
Figur 7-9 verschiedene Spektren der bei der elektrochirurgischen Behandlung unterschiedlicher Gewebe auftretenden Leuchterscheinung.
Figur 10 die Signaturen unterschiedlicher Gewebe nach Figur 9.

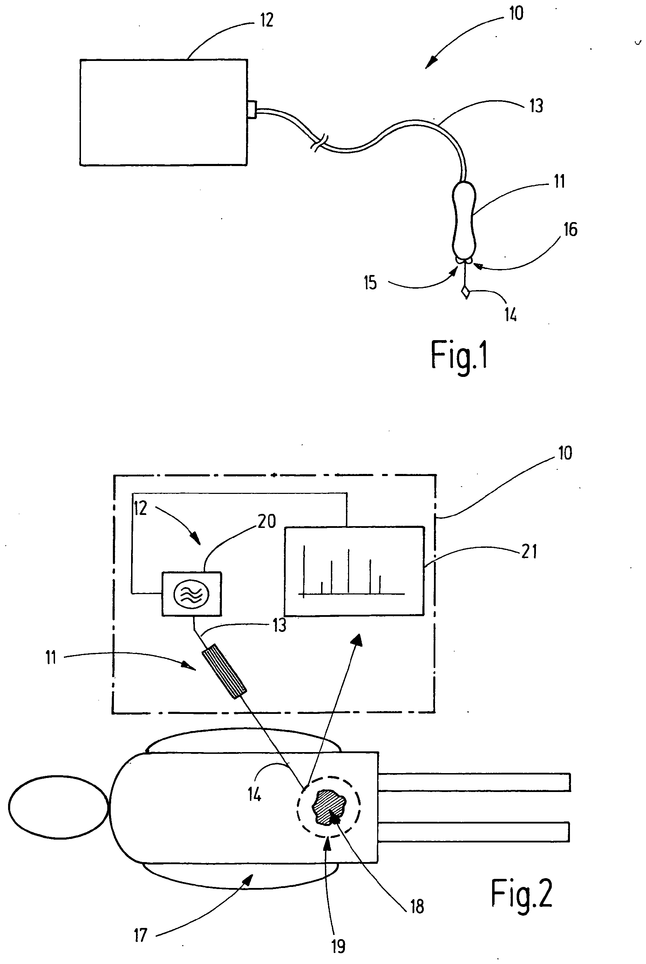

In Figur 1 ist eine chirurgische Einrichtung 10 veranschaulicht, zu der ein Instrument 11 und ein Gerät 12 zur Speisung desselben mit HF-Energie gehören. Das Gerät 12 und das Instrument 11 sind über eine Leitung 13 mit einander verbunden. Bei dem Gerät 11, das in Figur 1 symbolisch als Handgerät dargestellt ist, kann es sich um ein Elektroskalpell zur Anwendung in offenem Operationsgebiet oder auch um ein endoskopisches Instrument handeln. Es weist eine Elektrode 14 auf, die über die Leitung 13 mit HF-Energie beaufschlagbar ist. Außerdem sind an dem Instrument 11 oder auch von diesem gesondert mehrere Lichtaufnahmeeinrichtungen 15, 16 vorgesehen, die dazu dienen, an oder in der Nähe der Elektrode 14 entstehendes Licht zu empfangen. Die Lichtaufnahmeeinrichtungen 15, 16 sind über Lichtleiter, die sich durch die Leitung 13 erstrecken, mit dem Gerät 12 verbunden. Es ist möglich, eine Lichtverarbeitungseinrichtung in dem Instrument 11 anzuordnen, die Merkmale des Lichts charakterisierende Signale an das Gerät 12 liefert. Dies kann drahtlos oder auch über die Leitung 13 erfolgen.

Figur 2 veranschaulicht die Anwendung der Einrichtung 10 an einem Patienten 17 in schematischer Weise. Dieser weist einen Tumor 18 auf, der entlang einer Resektionslinie 19 im gesunden Gewebe zu entfernen ist.

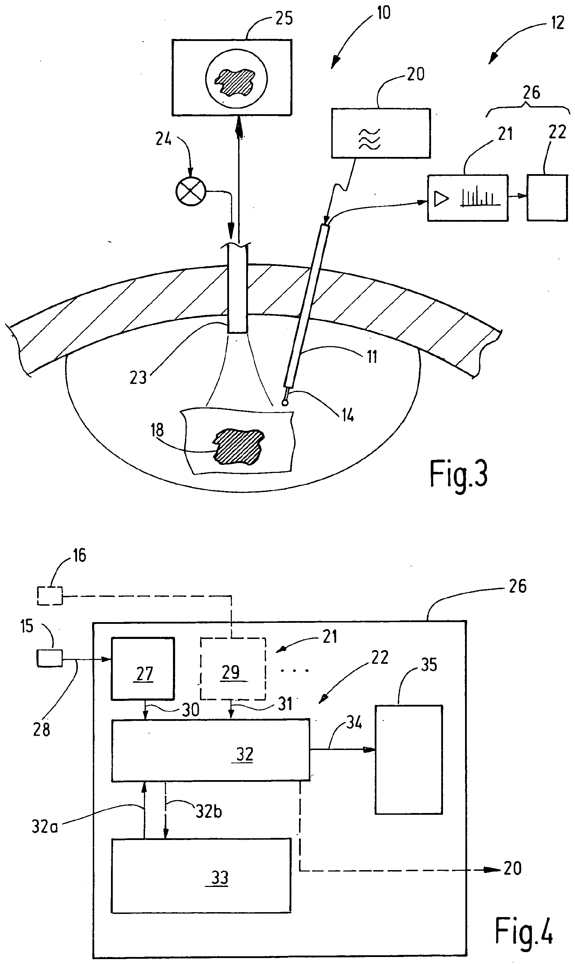

Das Gerät 12 enthält einen HF-Generator 20, der HF-Leistung kontrolliert bereitstellt. Außerdem enthält die Einrichtung 10 eine Analyseeinrichtung 21 zur Analyse des an der Elektrode 14 bei Auftreten eines HF-Funkens erzeugten Lichts. Die Analyseeinrichtung 21 kann z.B. eine Spektralzerlegung des von einem HF-Funken an der Elektrode 14 erzeugten gesamten Spektrums oder eines Teilspektrums vornehmen und in mindestens ein elektrisches Signal umsetzen. Figur 3 veranschaulicht dies nochmals am Beispiel einer schematisch dargestellten Operationssituation mit einem laparoskopischen Instrument 11. Dieses wird einerseits mit HF-Leistung gespeist und liefert andererseits Signale an die Analyseeinrichtung 21, die das aufgenommene Licht zerlegt und mindestens ein entsprechendes elektrisches Signal erzeugt, welches das Spektrum oder Teile desselben kennzeichnet.

Eine nachgeschaltete Diskriminatoreinrichtung 22 erhält von der Analyseeinrichtung 21 das Signal, das mindestens ein Merkmal des aufgenommenen Lichts kennzeichnet. Ein solches Merkmal ist eine Signatur, die aus den Intensitäten mehrerer Wellenlängen oder Teilspektren berechnet ist. Durch Vergleich mit abgespeicherten Vergleichswerten kann die Diskriminatoreinrichtung 22 ein Signal erzeugen, das die Art des Gewebes kennzeichnet, mit dem der HF-Funke in Berührung steht.

Die Einrichtung 10 kann weitere Elemente umfassen. Beispielhaft ist in Figur 3 dazu eine laparoskopische Kamera 23 dargestellt, die eine Lichtquelle 24 zur Beleuchtung des Operationsfelds umfasst und an einen Monitor 25 angeschlossen ist, über den der Chirurg das Operationsfeld beobachten kann.

Der grundsätzliche Aufbau der aus der Analyseeinrichtung 21 und der Diskriminatoreinrichtung 22 gebildeten Gewebeerkennungseinheit 26 ist in Figur 4 veranschaulicht. Diese Gewebeerkennungseinheit 26 kann vollständig innerhalb des Instruments 11, vollständig innerhalb des Geräts 12 oder auf das Instrument 11 und das Gerät 12 aufgeteilt realisiert sein. Beispielsweise kann die Analyseeinrichtung 21 innerhalb des Instruments 11 und die Diskriminatoreinrichtung 22 innerhalb des Geräts 12 angeordnet sein.

Zu der Analyseeinrichtung 21 gehört mindestens ein Lichtanalysator 27, der an seinem Eingang 28 Licht erhält, das von dem HF-Funken ausgeht, der zumindest zeitweilig an der Elektrode 14 brennt. Der Lichtanalysator 27 weist Mittel zur Erfassung spektraler Komponenten des aufgenommenen Lichts auf. Solche Mittel können ein oder mehrere schmalbandige Filter oder sonstige Mittel zur spektralen Zerlegung eines Lichtspektrums (Lichtzerlegevorrichtung) sein, wie beispielsweise ein Prisma zur Beleuchtung eines Fotodiodenfeldes, eines Kamerachips oder sonstige geeignete Mittel. Da mehrere Lichtaufnahmeeinrichtungen 15, 16 usw. vorhanden sind, sind entsprechend mehrere Lichtanalysatoren 27, 29 vorgesehen. Andere technische Lösungen zur Filterung spezifischer Wellenlängen können optische Bauelemente, wie beispielsweise Kollimator, Linsensystem, Fotodiodenarray und Auswerteeinrichtung umfassen. Diese Bauelemente können in integrierter Form beispielsweise in Wellenleiter-Strukturen oder sonstigen Filterschichten Anwendung finden.

Jeder Lichtanalysator 27, 29 liefert an seinem jeweiligen Ausgang 30, 31 ein oder mehrere vorzugsweise elektrische Signale, die jeweils ein oder mehrere Eigenschaften des mit den Aufnahmeeinrichtungen 15, 16 aufgenommenen Lichts kennzeichnen. Eigenschaften können z.B. die Intensitäten des Lichts bei einer oder bei mehreren Wellenlängen sein. Diese Eigenschaften können Lichtmerkmale sein oder als Grundlage zur Ermittlung von Lichtmerkmalen dienen.

Die Signale werden an die Diskriminatoreinrichtung 22 geliefert. Diese Diskriminatoreinrichtung umfasst Mittel bspw. in Form einer Verarbeitungseinrichtung 32, bspw. in

Gestalt eines Rechners. Die Verarbeitungseinrichtung 32 ist dazu vorgesehen und eingerichtet, die über die Ausgänge 30, 31 erhaltenen Signale, die Lichtmerkmale des aufgenommenen Lichts sind oder aus denen sie Lichtmerkmale ermittelt (z.B. errechnet), mit vorhandenen Mustern oder Referenzsignalen zu vergleichen, um den Lichtmerkmalen Gewebemerkmale zuzuordnen. Diese Zuordnung kann zur Gewebeunterscheidung herangezogen werden.

Die Diskriminatoreinrichtung 22 ist dazu über einen Kanal 32a mit einer Speichereinrichtung 33 verbunden. In dieser sind die Lichtmerkmale mehrerer Gewebetypen abgespeichert. Die Diskriminatoreinrichtung 22 ruft über den Kanal 32a Lichtmerkmale verschiedener Gewebetypen ab und vergleicht die zu verschiedenen Geweben oder Gewebetypen gehörigen abgespeicherten Lichtmerkmale mit den von den Lichtanalysatoren 27, 29 erhaltenen oder aus deren Signalen ermittelten Lichtmerkmalen. Sie ordnet dann den von den Lichtanalysatoren 27, 29 empfangenen Lichtmerkmalen dasjenige Gewebe zu, dessen abgespeicherte Merkmale am besten mit den empfangenen Merkmalen übereinstimmen.

Auf dieser Basis kann die Verarbeitungseinrichtung 32 an einem Ausgang 34 ein Signal abgeben, das zum Beispiel an eine Warneinrichtung 35 oder eine sonstige Signalisierungseinrichtung gegeben werden kann. Die Warneinrichtung 35 kann bspw. immer dann aktiviert werden, wenn die Verarbeitungseinrichtung 32 das Vorhandensein eines bestimmten Gewebetyps feststellt. Alternativ kann die Warneinrichtung 35 immer dann aktiviert werden, wenn die Verarbeitungseinrichtung 32 das Nichtvorhandensein eines bestimmten Gewebetyps feststellt. Das von der Warneinrichtung 35 abgegebene Signal kann ein optisches, akustisches oder sonstiges Signal sein, das vom Operateur wahrnehmbar ist.

Alternativ oder zusätzlich kann die Verarbeitungseinrichtung 32 ein Signal an den HF-Generator 20 geben, wie in Figur 4 durch eine gestrichelte Linie dargestellt ist. Beispielsweise kann der HF-Generator 20 dadurch in seiner Leistung oder hinsichtlich sonstiger Größen (z.B. dem Crestfaktor, dem Innenwiderstand) beeinflusst, geregelt oder abgeschaltet werden.

Die in Figur 4 veranschaulichte Speichereinrichtung 33 kann Lichtmerkmale oder Lichtspektren bestimmter Gewebetypen bereithalten. Es ist auch möglich, dass die Verarbeitungseinrichtung 32, wie in Figur 4 durch einen gestrichelten Signalpfad 32b angedeutet ist, Lichtmerkmale des aktuell empfangenen Lichts ein- oder mehrmals an die Speichereinrichtung 33 übergibt, um diese Lichtmerkmale später als Referenz zu nutzen. Damit können patientenindividuelle Lichtmerkmale gespeichert werden.

Es ist in einem unspezifischen Arbeitsmodus auch möglich, das empfangene Licht ohne Vergleich oder Abgleich mit einer Datenbank bei der Schnittführung fortwährend auf Konstanz der Lichtmerkmale zu untersuchen. Weichen aktuelle Lichtmerkmale plötzlich von zuvor ermittelten Lichtmerkmalen ab, kann dies gemeldet werden. Der Operateur kann daran erkennen, dass der Gewebetyp gewechselt hat, der mit dem Funken in Kontakt steht.

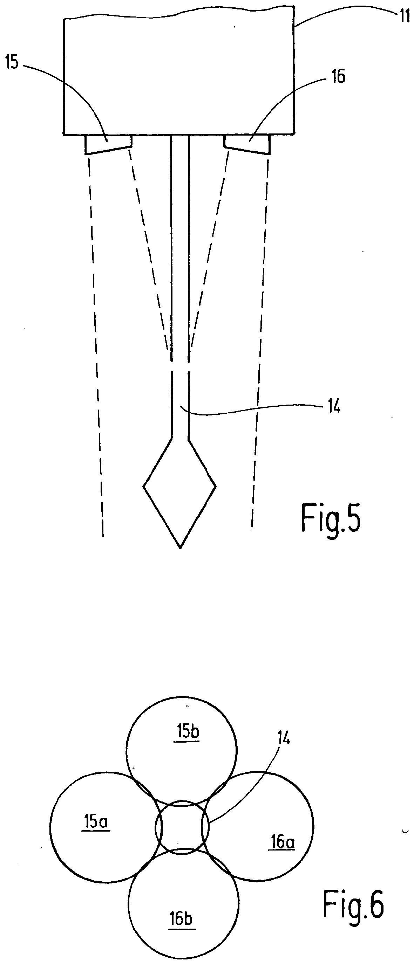

Figur 5 veranschaulicht das Instrument 11 schematisch. Wie ersichtlich, können die Lichtaufnahmeeinrichtungen 15, 16 mit unterschiedlicher Ausrichtung auf die Elektrode 14 schauend ausgerichtet sein. In einem nicht dargestellten Ausführungsbeispiel kann die Ausrichtung der Lichtaufnahmeeinrichtung 15, 16 durch eine andere Möglichkeit, beispielsweise durch eine Sammellinse, eine Kugellinse oder andere optisch wirkende Mittel, zur Maximierung der Lichtintensität erfolgen.

Figur 6 veranschaulicht einander mehr oder weniger überlappende Empfindlichkeitsbereiche 15a, 15b, 16a, 16b von insgesamt vier Lichtaufnahmeeinrichtungen. Die Anzahl der Lichtaufnahmeeinrichtungen kann jedoch auch größer oder kleiner sein.

Die insoweit beschriebene chirurgische Einrichtung 10 arbeitet wie folgt:

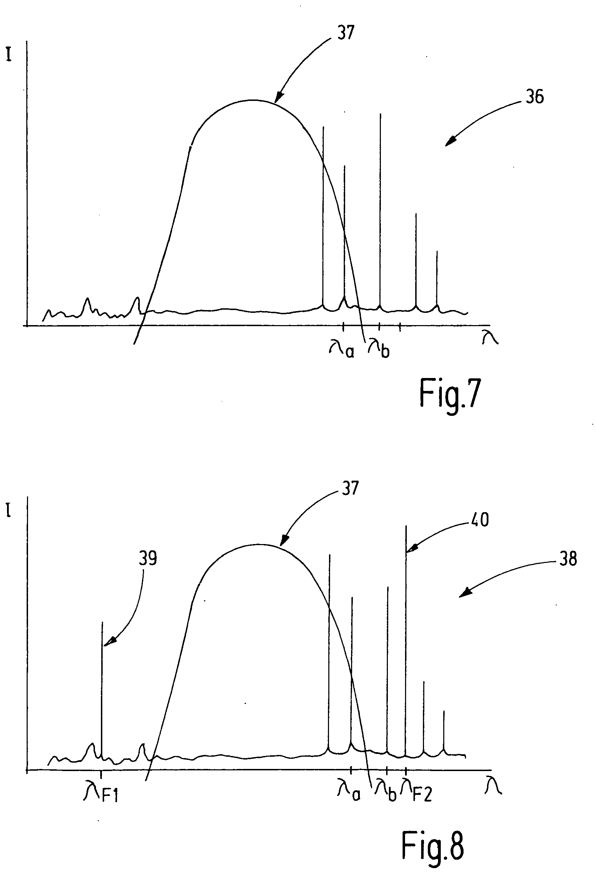

In Figur 7 ist schematisch ein Spektrum 36 veranschaulicht, das von einem HF-Funken ausgeht, der an der Elektrode 14 in einem gesunden biologischen Gewebe entsteht. Das Spektrum 36 ist näherungsweise ein Linienspektrum. Überlagert ist ein Spektrum 37 der Operationsfeldbeleuchtung. Es wird nun angenommen, dass der Patient nach Figur 2 mit der erfindungsgemäßen Einrichtung 10 einer Tumorresektion unterzogen werden soll. Er erhält dazu eine Injektion oder sonstige Gabe einer Markersubstanz, die eine tumorspezifische chemophore Gruppe, d.h. ein Tumorantigen aufweist, die mit einer Labelsubstanz verbunden ist. Die Labelsubstanz ist bspw. ein physiologisch unbedenkliches sonst im Körper nicht vorhandenes Metall oder ein sonstiger sonst im Körper nicht oder kaum vorhandener Stoff. Dieser zeichnet sich dadurch aus, dass er im Plasma auf einer oder mehreren charakteristischen Längenwellen λ_{F1}, λ_{F2} leuchtet, die vorzugsweise außerhalb des Spektrums 37 der Operationsfeldbeleuchtung liegen. Derartige Spektrallinien fehlen in dem Spektrum nach Figur 7.

Figur 8 veranschaulicht das Spektrum 38 des Tumorgewebes mit zwei Spektrallinien 39, 40, die von der Markersubstanz hervorgerufen werden. Je nach Selektivität des gewählten Tumormarkers kann die Diskriminatoreinrichtung sehr sicher erkennen, wann der HF-Funken das Tumorgewebe berührt. Dazu ist als Lichtmerkmal in dem Speicher 33 ein Intensitätsgrenzwert für die Wellenlänge λ_{F1} und/ oder die Wellenlänge λ_{F2} abgespeichert. Die Diskriminatoreinrichtung 22 unterscheidet nun gesundes Gewebe von krankhaftem Gewebe, indem die Verarbeitungseinrichtung 32 die aufgenommene Intensität bei den Wellenlängen λ_{F1} und/oder λ_{F2} mit dem in der Speichereinrichtung 33 abgespeicherten Grenzwerten vergleicht. Sind die Grenzwerte überschritten, erkennt die Diskriminatoreinrichtung 22 den Schnitt im kranken Gewebe und kann dazu ein entsprechendes Signal abgeben. Wird dieser Vergleich separat für das von den Lichtaufnahmeeinrichtungen 15, 16 aufgenommenen Licht durchgeführt, kann außerdem ermittelt und signalisiert werden, an welcher Seite oder Stelle des HF-Skalpells krankhaftes Gewebe ansteht.

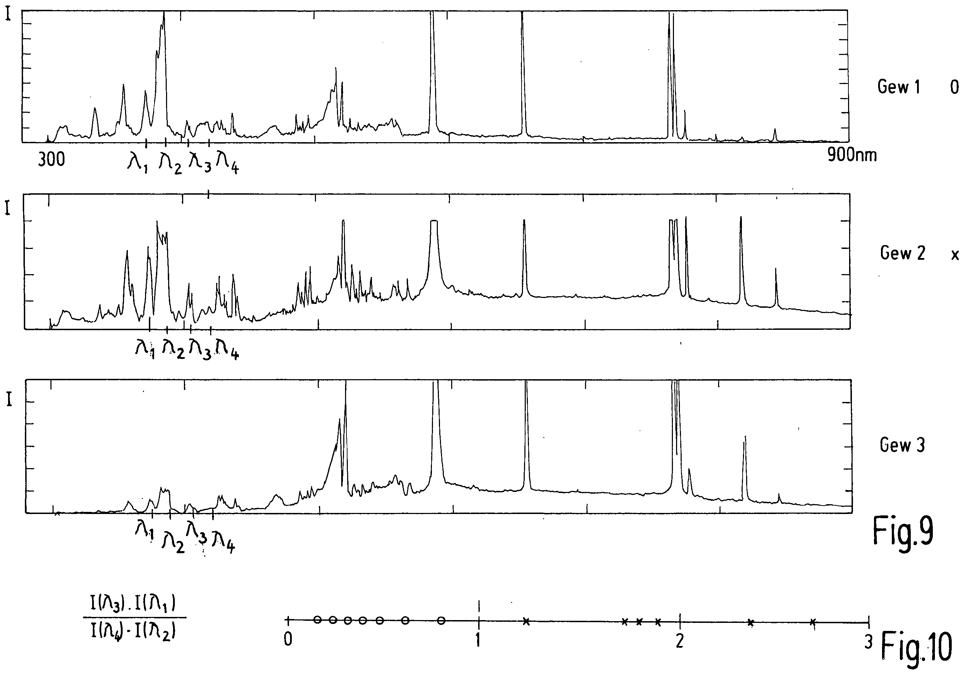

Aus den Figuren 9 und 10 geht die Arbeitsweise der Einrichtung 10 hervor, wenn ohne Markersubstanz gearbeitet werden soll. In Figur 9 sind die typischen Spektren veranschaulicht, die entstehen, wenn drei verschiedene Gewebearten GEW 1, GEW2, GEW3 (Leber, Niere, Muskel) mit einem HF-Funken in Berührung kommen. Die Spektren der drei Gewebetypen GW1, GW2, GW3 unterscheiden sich in einzelnen Merkmalen wie bspw. den Intensitäten der Wellenlängen λ_{F1} bis λ_{F4}. Die Gewebeerkennung bzw. Gewebeunterscheidung auf Basis der Lichtmerkmale dieser drei Spektren ist möglich. Die Diskriminatoreinrichtung 22 kann dazu auf verschiedenste Algorithmen wie bspw. Mustererkennung oder auch den Vergleich von Einzelintensitäten bspw. bei der Wellenlänge λ_{F2} zurückgreifen. Damit ist eine Unterscheidung der Gewebetypen GW1 und GW3 möglich.

Bei einer bevorzugten Ausführungsform wird aus den einzelnen Lichtmerkmalen bspw. in Gestalt der Intensitäten von vier Wellenlängen λ_{F1} bis λ_{F4} eine Signatur als kennzeichenkräftiges Lichtmerkmal berechnet. Dies ist in Figur 10 veranschaulicht. Als Beispiel wird die Signatur hier als Quotient aus Produkten der Intensitäten bei verschiedenen Wellenlängen berechnet. Es zeigt sich, dass die so berechneten Signaturen jeweils zwischen 0 und 3 liegen und zwar auch bei großen Versuchsserien mit Geweben von verschiedenen Patienten. Es zeigt sich weiter, dass die Signaturen des Gewebetyps GW2 oberhalb einer bestimmten Grenze, hier 1, liegen, während die Signaturen des Gewebetyps GW1 unterhalb dieser Grenze liegen.

Es versteht sich, dass zur Diskriminierung anderer Gewebe und Gewebetypen andere Lichtmerkmale, d.h. die Intensitäten anderer Wellenlängen auch in größerer oder geringer Zahl, und/oder andere Signaturen, die auf anderen Berechnungsformeln beruhen, genutzt werden können.

Alle oben genannten Varianten zur Erfassung oder Berechnung von Lichtmerkmalen, Z.B. Signaturen, können auch bei chirurgischen Instrumenten angewendet werden, bei denen ein Plasmastrahl auf das biologische Gewebe einwirkt. Vorzugsweise handelt es sich dabei um ein nichtthermisches Plasma, bei dem die Elektronentemperatur von der Plasmatemperatur abweicht.

Die erfindungsgemäße chirurgische Einrichtung 10 gibt dem Chirurgen ein Mittel in die Hand, während der Operation bei der chirurgischen Einwirkung auf Gewebe durch eine Analyse des bei der HF-chirurgischen Einwirkung erzeugten Lichts eine Information darüber zu erhalten, welcher Art das gerade an der HF-Elektrode 14 vorhandene Gewebe ist. Er kann dadurch verschiedene Gewebearten (Muskel, Fett, Blut, Organ) unterscheiden. Ebenso ist es möglich, eine Unterscheidung zwischen gesundem und krankhaftem Gewebe zu treffen. Durch die automatisierte schnelle Lichtanalyse des im HF-Funken oder im vorzugsweise nichtthermischen Plasma entstehenden Lichts kann die Operationssicherheit erhöht werden. Insbesondere ist es in vielen Fällen möglich, R0-Resektionen zu erzielen, die mit herkömmlichen Gerätschaften nicht zu erreichen sind.

### Bezugszeichenliste:

- 10: Chirurgische Einrichtung
- 11: Instrument
- 12: Gerät
- 13: Leitung
- 14: Elektrode
- 15, 16: Lichtaufnahmeeinrichtungen
- 17: Patient
- 18: Tumor
- 19: Resektionslinie
- 20: HF-Generator
- 21: Analyseeinrichtung
- 22: Diskriminatoreinrichtung
- 23: Kamera
- 24: Lichtquelle
- 25: Monitor
- 26: Gewebeerkennungseinheit
- 27, 29: Lichtanalysator
- 28: Eingang
- 30, 31: Ausgang
- 32: Mittel, Verarbeitungseinrichtung
- 33: Speichereinrichtung
- 34: Ausgang
- 35: Warneinrichtung
- 15a, b, 16a, b: Empfindlichkeits- / Lichtaufnahmebereiche
- 36: Spektrum des gesunden Gewebes
- 37: Spektrum der Operationsfeldbeleuchtung
- 38: Spektrum des kranken Gewebes
- 39, 40: Spektrallinien der Markersubstanz
- Gew 1: erster Gewebetyp
- Gew 2: zweiter Gewebetyp
- Gew 3: dritter Gewebetyp

## Patentansprüche

1. Chirurgische Einrichtung (10) mit:
einem Instrument (11) zur Einwirkung auf ein biologisches Gewebe unter Erzeugung eines Plasmas mit einer Lichterscheinung,
einem Gerät (12) zur Speisung des Instruments (11) mit elektrischer HF-Leistung,
wobei das Instrument (11) mindestens eine HF-Elektrode (14) aufweist, die über eine Leitung (13) mit dem Gerät (12) verbunden ist, um mit HF-Leistung gespeist zu werden, wobei sich ein HF-Funke zwischen der mindestens einen HF-Elektrode (14) und dem biologischen Gewebe ausbildet,
mit einer Lichtaufnahmeeinrichtung (15), die über einen Lichtleiter, der sich durch die Leitung (13) erstreckt, mit dem Gerät (12) verbunden ist,
einer Analyseeinrichtung (21) zur Bestimmung von Lichtmerkmalen des durch die Einwirkung auf das biologische Gewebe erzeugten Lichts, wobei die Analyseeinrichtung (21) eine Verarbeitungseinrichtung (32) zur Ermittlung von Lichtmerkmalen aufweist,
wobei die von der Verarbeitungseinrichtung (32) ermittelten Lichtmerkmale Signaturen sind, die aus den Intensitäten mehrerer Wellenlängen oder Teilspektren berechnet sind, sowie mit
einer Diskriminatoreinrichtung (22) zur Zuordnung von Lichtmerkmalen des bei der Einwirkung erzeugten Lichts zu Gewebemerkmalen des der Einwirkung unterliegenden biologischen Gewebes
**dadurch gekennzeichnet,**
**dass** das Plasma eine Plasmatemperatur aufweist, die so niedrig gehalten ist, dass in dem Plasma verdampfte Teile des biologischen Gewebes nicht vollständig dissoziiert sind,
**dass** an dem Instrument (11) mehrere Lichtaufnahmeeinrichtungen (15, 16) angeordnet sind, die jeweils auf einen Teilbereich des die Lichterscheinung enthaltenden Bereichs ausgerichtet sind und vorwiegend Licht aus unterschiedlichen Abschnitten des die Lichterscheinung enthaltenden Bereichs (15a, 16a, 15b, 16b) aufnehmen, um Richtungsinformation zu gewinnen, und
**dass** zur Unterscheidung zwischen gesundem Gewebe und pathologischem Gewebe jede Signatur aus Intensitätsverhältnissen ausgewählter, für verdampfte Teile des biologischen Gewebes kennzeichnender Spektrallinien berechnet ist.

2. Einrichtung nach Anspruch 1, **dadurch gekennzeichnet, dass** die Analyseeinrichtung (21) eine Filtereinrichtung zur selektiven Erfassung wenigstens zweier spektraler Komponenten der Lichterscheinung aufweist.

3. Einrichtung nach einem der vorstehenden Ansprüche, **dadurch gekennzeichnet, dass** dem Instrument (11) eine Operationsfeldbeleuchtungseinrichtung (24) zugeordnet ist.

4. Einrichtung nach Anspruch 3, **dadurch gekennzeichnet, dass** eine von der Analyseeinrichtung (21) zu erfassende spektrale Komponente (A) außerhalb des Lichtspektrums der Operationsfeldbeleuchtung (24) festgelegt ist.

## Claims

1. Surgical device (10) with:
an instrument (11) for acting on a biological tissue by creation of a plasma with a light phenomenon,
an appliance (12) for supplying the instrument (11) with electrical HF power,
wherein the instrument (11) has at least one HF electrode (14) which is connected to the appliance (12) via a line (13) in order to be supplied with HF power, wherein an HF spark forms between the at least one HF electrode (14) and the biological tissue,
with a light-receiving device (15) which is connected to the appliance (12) via a light conductor which extends through the line (13),
an analysis device (21) for determining light features of the light created by the action on the biological tissue, wherein the analysis device (21) has a processing device (32) for determining light features,
wherein the light features determined by the processing device (32) are signatures which are calculated from the intensities of several wavelengths or part spectra, and with
a discriminator device (22) for assigning light features of the light created during the action to tissue features of the biological tissue subjected to the action,
**characterized in that**
the plasma has a plasma temperature which is kept so low that parts of the biological tissue which have vaporized in the plasma are not completely dissociated,
that several light-receiving devices (15, 16) are arranged on the instrument, which are each oriented at a partial region of the region containing the light phenomenon and mainly receive light from different portions of the region (15a, 16a, 15b, 16b) containing the light phenomenon, in order to obtain direction information, and
that to distinguish between healthy tissue and pathological tissue, each signature is calculated from intensity ratios of selected spectral lines characteristic of vaporized parts of the biological tissue.

2. Device according to claim 1, **characterized in that** the analysis device (2) has a filter device for selectively detecting at least two spectral components of the light phenomenon.

3. Device according to one of the preceding claims, **characterized in that** an operational field illumination device (24) is assigned to the instrument (11).

4. Device according to claim 3, **characterized in that** a spectral component (λ) to be detected by the analysis device (21) is established outside the light spectrum of the operational field illumination (24).

## Revendications

1. Dispositif chirurgical (10) comprenant :
un instrument (11) destiné à agir sur un tissu biologique en produisant un plasma avec une apparition de lumière,
un appareil (12) destiné à alimenter l'instrument (11) en puissance HF électrique,
l'instrument (11) présentant au moins une électrode HF (14) qui est reliée par une ligne (13) à l'appareil (12) afin d'être alimentée en puissance HF, une étincelle HF se formant entre l'électrode HF (14), au nombre d'au moins une, et le tissu biologique,
comprenant un dispositif de réception de lumière (15) qui est relié à l'appareil (12) par l'intermédiaire d'un conducteur de lumière qui s'étend dans la ligne (13),
un dispositif d'analyse (21) destiné à déterminer des caractéristiques de la lumière produite par l'action sur le tissu biologique, le dispositif d'analyse (21) présentant un dispositif de traitement (32) pour la détermination de caractéristiques de lumière,
les caractéristiques de lumière déterminées par le dispositif de traitement (32) étant des signatures qui sont calculées à partir des intensités de plusieurs longueurs d'ondes ou spectres partiels, et comprenant
un dispositif discriminateur (22) destiné à associer des caractéristiques de la lumière produite lors de l'action à des caractéristiques tissulaires du tissu biologique soumis à l'action,
**caractérisé**
**en ce que** le plasma présente une température de plasma qui est maintenue si faible que des parties du tissu biologique qui se sont vaporisées dans le plasma ne sont pas complètement dissociées,
**en ce que** sont prévus sur l'instrument (11), plusieurs dispositifs de réception de lumière (15, 16) qui sont orientés respectivement sur une zone partielle de la zone contenant l'apparition de lumière et reçoivent principalement de la lumière provenant de différentes portions de la zone (15a, 16a, 15b, 16b) contenant l'apparition de lumière, afin d'obtenir des informations directionnelles, et
**en ce que** pour la distinction entre le tissu sain et le tissu pathologique, chaque signature est calculée à partir de rapports d'intensité de raies spectrales sélectionnées, caractéristiques de parties vaporisées du tissu biologique.

2. Dispositif selon la revendication 1, **caractérisé en ce que** le dispositif d'analyse (21) présente un dispositif de filtre destiné au recueil sélectif d'au moins deux composantes spectrales de l'apparition de lumière.

3. Dispositif selon l'une des revendications précédentes, **caractérisé en ce qu'**un dispositif d'éclairage de champ opératoire (24) est associé à l'instrument (11).

4. Dispositif selon la revendication 3, **caractérisé en ce qu'**une composante spectrale (A) à recueillir par le dispositif d'analyse (21) est définie à l'extérieur du spectre lumineux de l'éclairage de champ opératoire (24).
